(19) Europäisches Patentamt
European Patent Office
Office européen des brevets

(11) **EP 2 124 847 B1**

(12) **EUROPEAN PATENT SPECIFICATION**

(45) Date of publication and mention
of the grant of the patent:
**16.05.2012 Bulletin 2012/20**

(21) Application number: **07736720.9**

(22) Date of filing: **23.03.2007**

(51) Int Cl.:
**A61F 2/90** (2006.01)    **A61F 2/06** (2006.01)

(86) International application number:
**PCT/IT2007/000216**

(87) International publication number:
**WO 2008/117315 (02.10.2008 Gazette 2008/40)**

(54) **ENDOLUMINAL PROSTHESIS**

ENDOLUMINALE PROTHESE

PROTHÈSE ENDOLUMINALE

(84) Designated Contracting States:
**AT BE BG CH CY CZ DE DK EE ES FI FR GB GR HU IE IS IT LI LT LU LV MC MT NL PL PT RO SE SI SK TR**

(43) Date of publication of application:
**02.12.2009 Bulletin 2009/49**

(73) Proprietor: **Invatec Technology Center GMBH CH-8500 Frauenfeld (CH)**

(72) Inventors:
• **VENTURELLI Andrea**
**I-25060 CONCESIO**
**(Brescia) (IT)**

• **SCHAFFNER, Silvio**
**CH-8267 BERLINGEN (CH)**

(74) Representative: **Zimmermann & Partner**
**Postfach 330 920**
**80069 München (DE)**

(56) References cited:
**EP-A- 0 701 800     EP-A- 1 834 606**
**WO-A-2005/058206     WO-A-2006/034062**
**WO-A-2007/013102     US-A1- 2003 045 926**

**Description**

Forming the object of the present invention is an endoluminal prosthesis, or stent, for use in passages or ducts of living bodies, above all the human body. Such endoluminal prosthesis can be used, for example, for restoring the passage sin blood vessels reduced or obstructed by pathological phenomena such as a stenosis. Such endoluminal prosthesis can also be used in bile ducts or other similar organs.

The present:invention refers to a type of endoluminal prosthesis which is positioned in a radially contracted state inside the selected duct. Once in place, the prosthesis is brought into expanded state, until it reaches the suitable size for the duct.

Documents WO2006/034062 and WO2006/058206 disclose devices having anchors which can be used to secure tubes within a digestive truck. Each anchor connected with the near one with a joining member. Document WO2008/030488 and WO9413268 disclose stents with a suture interwoven in a over-and-under pattern through the struts.

. For some types of endoluminal prostheses called "balloon-expandable", the expansion step is usually completed by applying a radial pressure from the interior, Such pressure is generally applied by means of an element, called ball, which, can be radially expanded by means of the insertion of a fluid under pressure.

. Such "balloon-expandable" prostheses are made, for example, with stainless steel or with chrome-cobalt alloys.

. Other endoluminal prosthesis types called "self-expandable", are made so to spontaneously take on an expanded configuration The expansion step is usually completed by releasing the prosthesis from a radial constriction.

Such "self-expandable" prosthesis are made, for example, of superelastic materials or with shape memory materials, such as Nitinol.

The known endoluminal prostheses or stents are generally formed by a succession of bands arranged next to each other in axial direction and connected to each other by means of bridges. The bands are radially contractible and expandable. In turn, the bridges are often elastic in the axial and circumferential direction.

. Due to this structure, above all thanks to the radially contractible and expandable bands, the stent is capable firstly of assuming a contracted configuration and an expanded configuration. Moreover the stent, due above all to the elastic bridges in the axial and circumferential direction, is capable of following all of the movements and deformations of the vessel during its operation life.

. These endoluminal prostheses, while satisfactory from many standpoints, in particular for their great flexibility and elasticity, which permit easily slipping the prosthesis in contracted state into narrow and tortuous passages, are in turn not sufficiently adapted, in the operating life, for supporting the continuous stress applied by the vessel walls.

. In particular, the stresses which are most dangerous for the prosthesis are the "fatigue" stresses, i.e. those stresses which derive from loads which can vary over time. Such stresses translate into a state of strain oscillating around an average value.

. In general, the fatigue stresses can lead a mechanical piece to failure or breaking, even if during the operation life a strain peak is never registered which exceeds the static breaking limit of the piece itself.

. In the specific case of the endoluminal prostheses or stents, the fatigue stresses become particularly dangerous for the bridges which join the bands together.

. Notwithstanding the severe tests to which the stents must be subjected in order to be used in the care of human patients, it is unfortunately possible that a bridge breaking occurs due to fatigue.

. The bridge breaking originates two stumps and two fracture surface. The two stumps, no longer connected with each other, are much less flexible than the entire bridge and are less adapted to follow the deformations of the vessel walls on which they lie.

. In addition, the two fracture surfaces do not have the characteristics of the other stent surfaces, suitably treated in the production step to come into contact with the vessel walls. Often, moreover, the fracture surface have sharp, if not cutting edges.

. It is therefore clear that the occurrence of a similar fracture translates into a dangerous stress of the vessel wall. Such stress is dangerous since it could soon lead, in the worst cases, to the perforation of the wall. In less serious cases, in the long term, it could lead to a local thickening of the wall, undoing the effect which had been originally intended by installing the stent.

. Stents of known type have a further problem. The implant time of the stent represents an acute step of stress of the vessel wall, which therefore requires a great support. A stent of traditional type, developed for optimising the support during this first acute step, then risks not ensuring good behaviour during the subsequent chronic step. In such step, in fact, the necessary support is widely reduced and an excessive quantity of metal inside the vessel risks representing a constant stress factor for the wall.

. The known stents, above all of "self-expandable" type finally have one other problem. During the release step inside the vessel, when the sheath which provides the radial constriction is pulled back, there is an elongation of the stent. Such elongation can cause on the one hand a longitudinal stress of the vessel, and on the other hand an actual jump ahead of the stent along the vessel. The jump ahead represents a big problem for the correct positioning of the stent itself.

. The operator which carries out the operation can in fact be fooled by this hard-to-predict behaviour of the stent. The search of the correct positioning of the stent can be made futile by the latter's jump ahead at the moment of the release.

. The object of the present invention is that of proposing an endoluminal prosthesis, which has structural and functional characteristics so to at least partially overcome the aforesaid drawbacks mentioned with reference to the prior art.

. In particular, a task of the present invention is that of proposing an endoluminal prosthesis which permits providing a greater support immediately after the implant and slowly reducing it during the operation life.

. In particular, a task of the present invention is that of proposing an endoluminal prosthesis, which drastically reduces the fatigue breaking.

. In particolare, a task of the present invention is that of proposing an endoluminal prosthesis which resolves the problem deriving from the elongation and from the consequent jump which occurs during the release step.

. Such object and such tasks are attained by means of an endoluminal prosthesis of the type described in claim 1.

. Further embodiments are described in the dependent claims.

. Further characteristics and advantages of the prosthesis according to the invention result from the following description of its preferred embodiments, given as indicative and non-limiting, with reference to the attached figures; wherein:

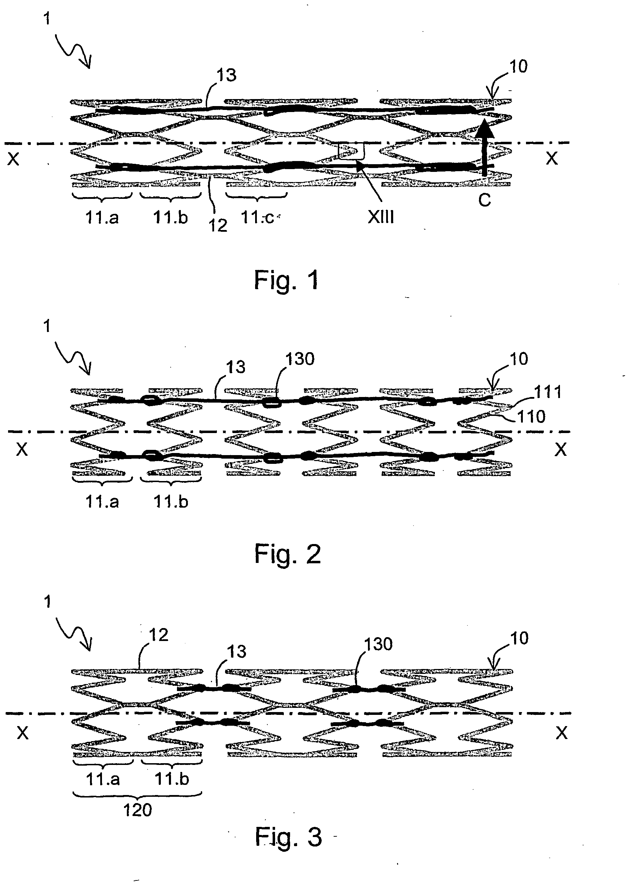

. Figure 1 schematically illustrate a stent in accordance with the invention;

. Figure 2 schematically illustrate another stent in accordance with the invention;

. Figure 3 schematically illustrate another stent in accordance with the invention;

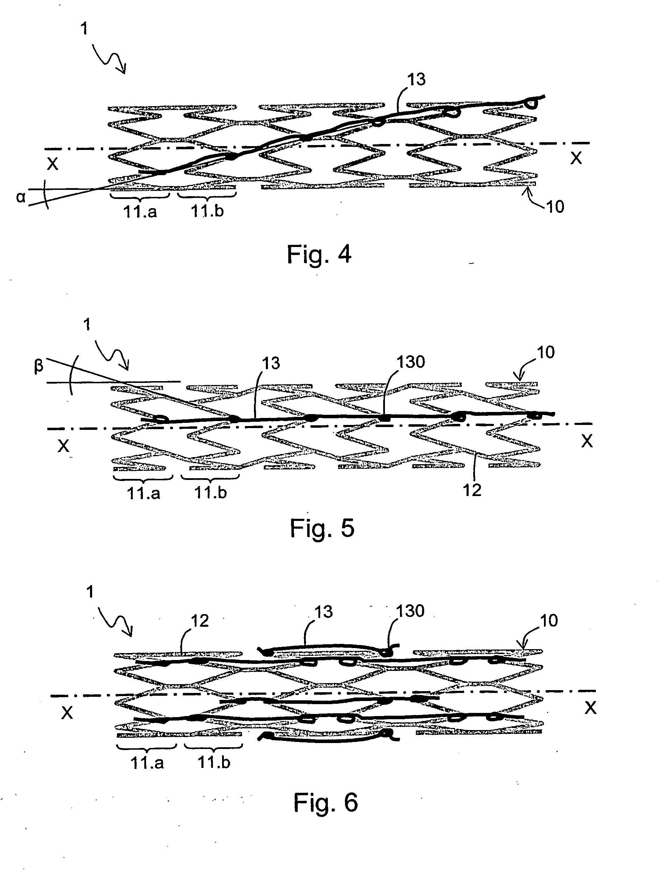

. Figure 4 schematically illustrate another stent in accordance with the invention;

. Figure 5 schematically illustrate another stent in accordance with the invention;

. Figure 6 schematically illustrate another stent in accordance with the invention;

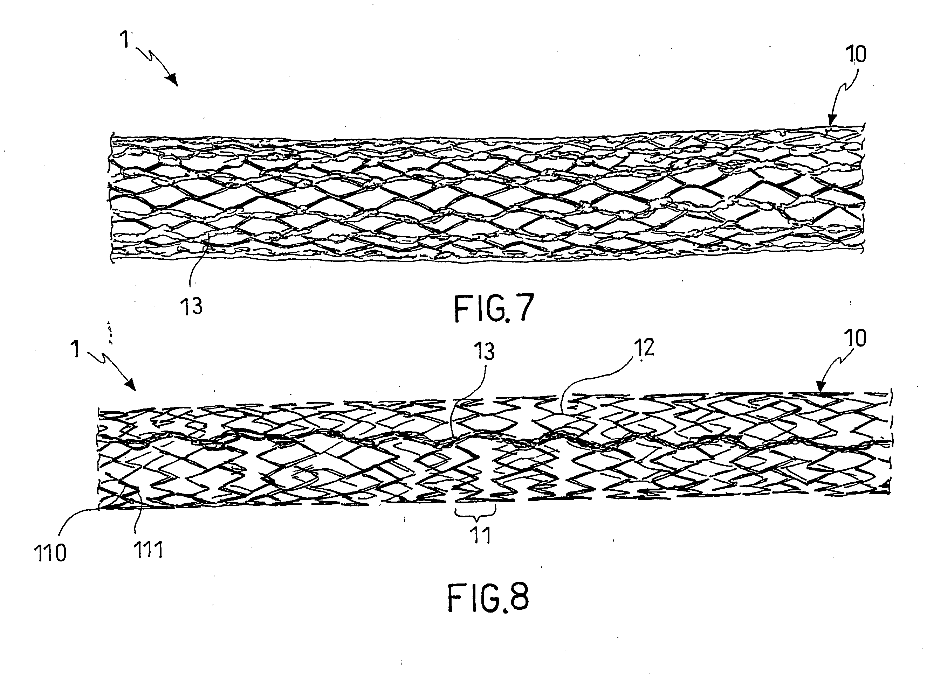

. Figure 7 illustrate, in perspective view, the central part of a stent similar to that of figure 1;

. Figure 8 illustrates, in perspective view, the central part of another stent in accordance with the invention;

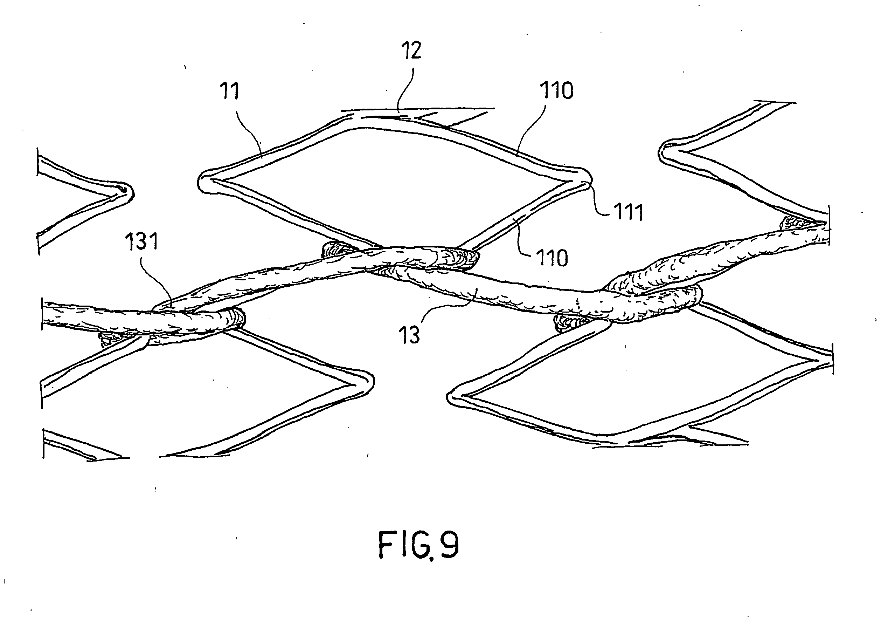

. Figure 9 illustrates a detail of the stent of figure 7 or 8;

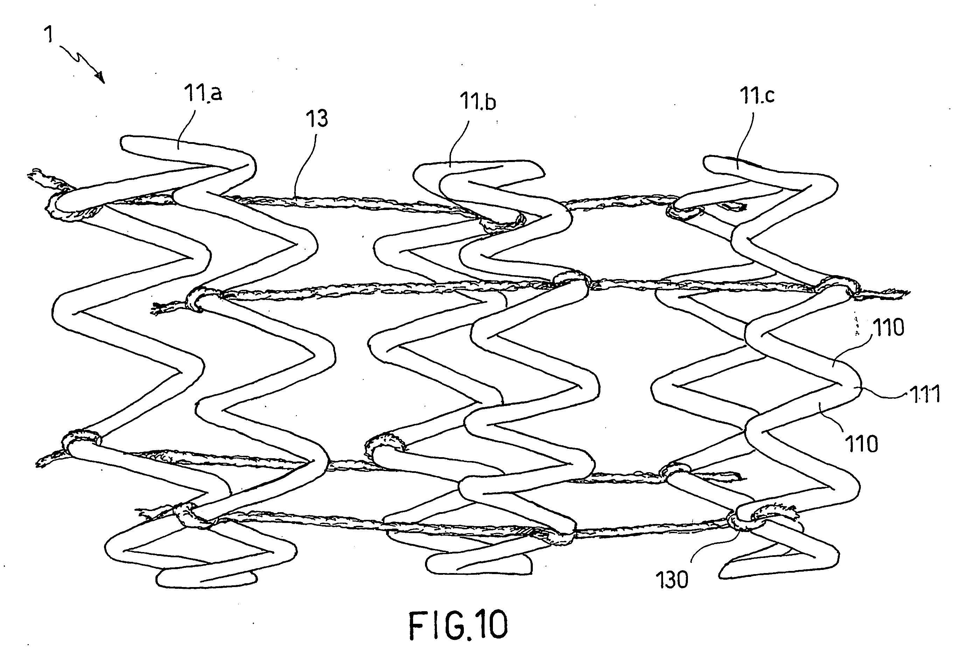

. Figure 10 illustrate, in perspective view, the central part of a stent similar to that of figure 2;

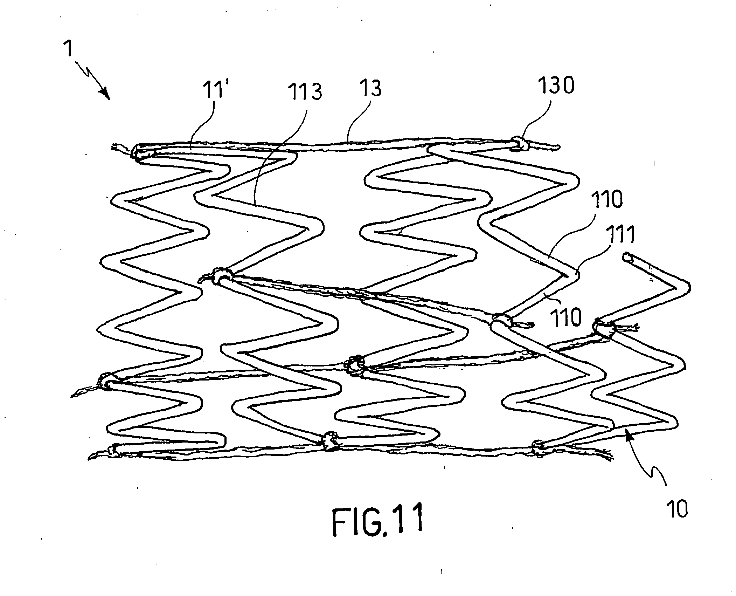

. Figure 11 illustrates, in perspective view, another stent in accordance with the invention;

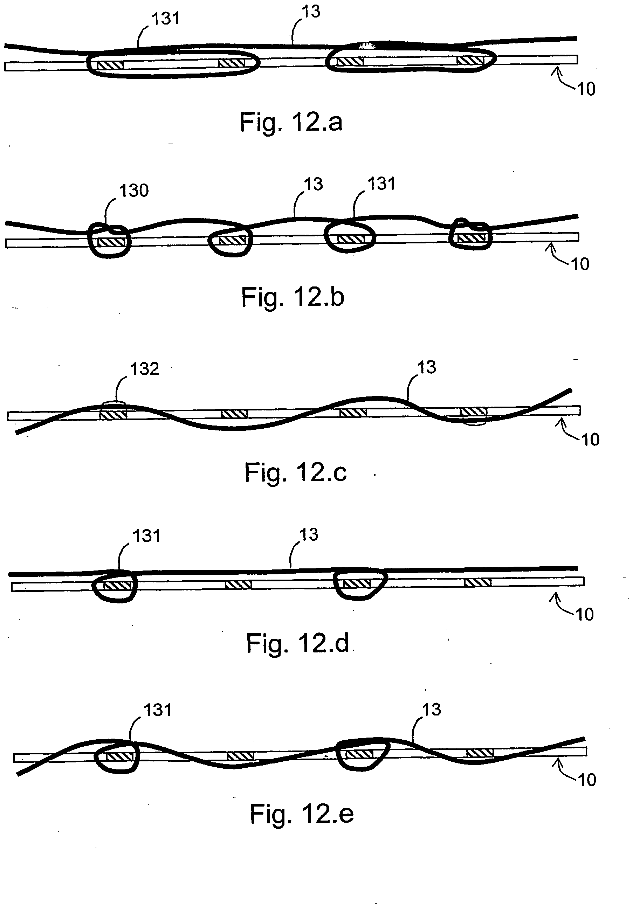

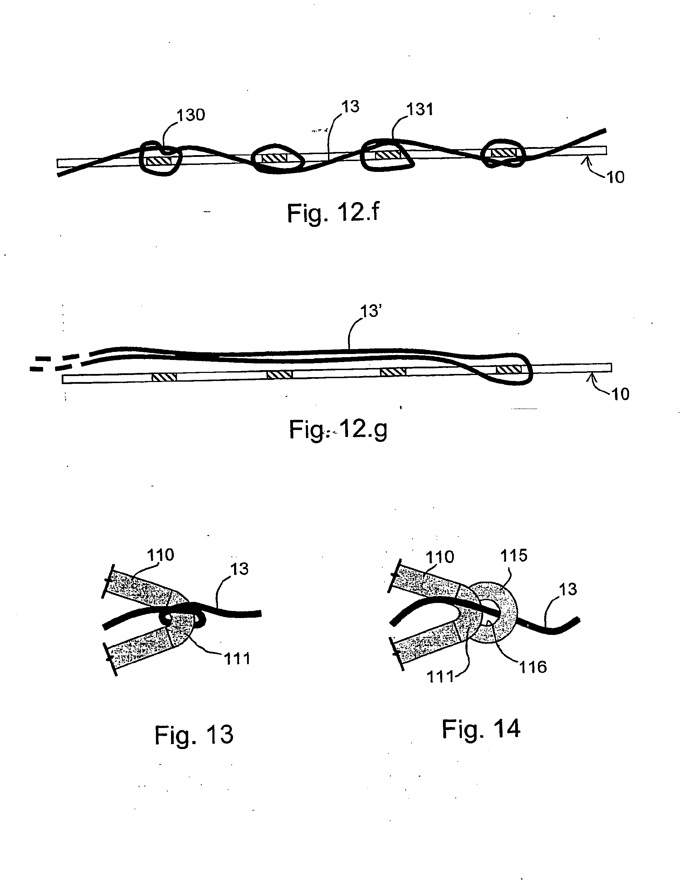

. Figures 12.a - 12.g schematically illustrate in detail some embodiments of the stent according to the present invention;

. Figure 13 schematically illustrates, a detail similar to the detail indicated with XIII in figure 1;

. Figure 14 schematically illustrates a variant of the detail of figure 13.

. With reference to the aforesaid figures, an endoluminal prosthesis or stent is indicated overall with 1. The stent 1 can be either of "balloon-expandable" or "self-expandable" type.

. In accordance with a general form of the present invention, the endoluminal prosthesis 1, comprises a tubular body 10 adapted to bring itself from a contracted condition to an expanded or partially expanded condition.

. With the term "contracted condition" it is intended a radially-compressed state of the stent 1, so to have a lower outer diameter and a lower radial size with respect to those of use.

. For example, the stent 1 is arranged in contracted condition when it is received or arranged on a transport and implant device (catheter) suitable to go through a duct or vessel up to the zone to be treated.

. For example, a stent of self-expandable type is arranged on a catheter and is contained in sheath which, by radially compressing the stent, keeps it in the contracted state.

. A stent of balloon-expandable type is arranged in contracted configuration on the deflated balloon of a catheter.

. With the term "expanded condition", it is intended a condition in which the stent 1 is radially enlarged, and in use comes into contact with the inner surface of the walls of a duct or vessel.

. For example, the stent 1 is arranged in an expanded condition when it is definitively placed in the zone to be treated of a duct or vessel.

. For example, in the case of a self-expandable stent, once the stent 1 is brought into place by means of the catheter, the sheath which radially compresses it is removed and the stent 1 spontaneously passes to its expanded condition.

. In the case of a balloon-expandable stent, on the other hand, once the stent 1 is brought into place by means of the catheter, the balloon is inflated. By pressing radially on the inside of the stent 1, the balloon brings the stent 1 to its expanded condition.

. The tubular body 10 of the stent 1 is developed along a longitudinal axis X-X.

. With "longitudinal axis" it is intended for example a symmetry axis of a cylindrical body or the axial direction of principal extension of a tubular body.

. Every direction parallel to the X-X axis of therefore defined as axial direction.

. As schematically indicated in figure 1, the tubular body 10 comprises a plurality of bands 11.a, 11.b, 11.c, etc. Such bands define paths which are preferably closed on each other. In the embodiments represented in the attached figures, when the stent 1 is in expanded condition, the bands 11 are developed along a substantially circumferential direction (indicated with C in figure 1).

. Moreover, in the stent 1 of the attached figures, the bands 11 assume serpentine form.

. With "serpentine band" it is intended a band which extends according to a zigzag course or backward-forward path around a prevalent extension-direction. In the case of the serpentine bands which form the stent 1 represented in the attached figures, the prevalent direction is that circumferential C around which the zigzag progression extends.

. Each of said serpentine bands 11 comprises arm portions, or arms 110, and loop portions, or loops 111, which connect two successive arms 110 to form the meandering path.

. In accordance with the embodiment schematically represented in figure 13, the arms 110 are substantially rectilinear and the loops 111 are substantially a circular crown sector.

. In accordance with another embodiment, the arms 110 are shaped along a curved line, for example 'S' shaped.

. At least one thread 13 connects at least two bands, for example two adjacent bands such as 11.a and 11.b, or two non-adjacent bands like 11.a and 11.c.

. With "thread" it is intended an elongated and extremely flexible element. Defining a proper axis of the thread, the characteristic dimensions of any cross section perpendicular to the proper axis are in general negligible with respect to the third dimension along the axis. The thread is composed of a single filament or, preferably, by a plurality of filaments assembled together. Where there is a plurality of filaments, they can be intertwined or twisted together so to remain assembled together. The thread can also comprise an outer covering.

. In general, the mechanical characteristics (stiffness and strength) of the thread are such to permit the same to react in a significant manner only with respect to a traction force along its axis. On the other hand, the reactions of the thread are in general negligible with respect to the other possible stresses: compression, twisting, flexion.

. The person skilled in the art will understand from the foregoing the differences between the thread as described and other elongated structures (bars, rods, staffs and the like) of comparable dimensions.

. From the foregoing, it can be deduced, for example, how the thread is an element characterised by a good knotting behaviour.

. The knotting behaviour can for example be expressed as a ratio between the inner diameter of a knot made with the thread, momentarily subjected to a determinate traction force, and the nominal diameter of the thread itself. A low ratio indicates a thread which is easy to knot (the knot closes well and easily holds). A thread with high ratio will be harder to manage (it is stiffer) and will produce knots which are easier to undo.

. In accordance with the embodiments of the stent 1 schematically represented in the figures 1-3 and 5-6, the thread 13 has an extension oriented in a substantially axial direction, substantially parallel to the axis X-X.

. In accordance with other embodiments, for example that represented schematically in figure 4, the thread 13 has a development oriented, in addition to in the axial direction, also in the circumferential direction, so to obtain a helical progression along the stent 1.

. In accordance with other embodiments of the stent 1 according to the invention (for example the embodiments represented in figures 1-2 and 4-6), two or more serpentine bands are connected with each other by means of a single thread portion 13.

. In accordance with several embodiments, the thread 13 is prevalently arranged on the outer surface of the stent 1. In other words, when the stent is situated inside a duct, most of the length of the thread 13 comes into contact with the inner wall of the duct itself.

. In accordance with several embodiments (see for example figure 2), the bands 11 of the stent 1 are exclusively connected with each other by threads 13.

. The threads 13 can connect two adjoining serpentine bands, for example 11.a and 11.b, or two not-immediately adjoining serpentine bands, for example 11.a and 11.c.

. In accordance with several embodiments (see for example figures 1, 3-6), the bands 11 of the stent 1 are connected to each other by bridges 12.

. The bridges 12, in a known manner, connect the loops 111 of two adjoining serpentine bands, for example 11.a and 11.b.

. There are some important differences between the bridges 12 and the thread 13. First, the bridges 12 are integral and made in one piece with the serpentine bands 11, while the thread is subsequently attached to the stent.

. Moreover, the thread is flexible and is capable of resisting only the traction forces applied along the X-X axis. On the other hand, the bridges are relatively rigid and are capable of offering resistance to all the forces (both traction and compression) applied along the X-X axis.

. Finally, the thread 13 is made with a different material than that employed for making the serpentine bands 11. On the other hand, the bridges 12 are necessarily made with the same material.

. The materials employed for the different structures (bands or serpentine bands 11, bridges 12 and thread 13) will be described in detail below.

. Advantageously, between adjacent serpentine bands, for example 11.a and 11.b, a plurality of threads 13 is provided.

. In accordance with the embodiment represented in figure 7, every single loop 111 of every single serpentine band, for example 11.b, is connected to the respective loop 111 of the adjacent serpentine band, for example 11.a or 11.c. The connections between adjacent loops can be obtained by means of a thread portion 13 or by means of a bridge 12.

. In accordance with the embodiment represented in figure 4, the thread portion has a slightly tilted direction with respect to the axial direction X-X of the tubular body 10. The direction of the thread 13 is for example tilted an angle equal to $\pm\alpha$ with respect to the axial direction X-X.

. Preferably, all of the threads 13 between two adjacent serpentine bands 11 are parallel to each other.

. In accordance with the embodiment schematically represented in figure 5, the bridges 12 also have a slightly tilted direction with respect to the axial direction X-X of the tubular body 10. The direction of the bridges 12 is for example tilted an angle equal to $\pm\beta$ with respect to the axial direction X-X.

. In particular, in the embodiment of figure 5, following the stent 1 longitudinally, for example going from a first proximal end to a second distal end of the stent, there are bridges 12 which are alternated with directions having opposite slopes (respectively $+\beta$ and $-\beta$) with respect to the axial direction X-X.

. In accordance with an embodiment schematically represented in figure 3, the stent comprises sections 120 comprising in turn several serpentine bands joined together, in a known manner, by bridges 12. The sections 120, on the other hand, are exclusively connected to each by threads 13 and not by bridges 12.

. In the particular embodiment schematised in figure 3, one can identify three sections 120, each one comprising two serpentine bands. In accordance with other possible embodiments, the number of sections 120 and/or serpentine bands for each section can be chosen differently, in consideration of specific needs..

. For example, the number of serpentine bands 11 for each section 120 can increase along the axis X-X from the proximal end towards the centre of the stent 1. Once the maximum number of serpentine bands 11 is reached in the central section 120, the number of serpentine bands for each section can once again decrease along the X-X axis from the centre of the stent towards the distal end.

. In the particular embodiment schematised in figure 6, it can be observed that the threads 13 attached to the stent 1 have different lengths. Each thread is applied so to cover the central portion of the stent 1. In this manner one obtains a quantity of threads 13 which increases along the axis X-X from the proximal end towards the centre of the stent 1. Once the maximum has been reached in the central portion, the number of threads 13 once again decreases along the X-X axis from the centre of the stent towards the distal end.

. In the particular embodiment schematised in figure 11, it can be observed that the bands of the stent are composed of curls 11' of a single, long helical serpentine band 113. In this case, the progression of the serpentine band 113 does not oscillate around a circumferential direction closed on itself but around a helix, for example cylindrical, which runs through the entire body 10 of the stent 1. The curls 11' created by the helical serpentine band 113 diverge little from the progression of the serpentine bands 11 described above and thus they substantially maintain the circumferential direction, swaying little from it.

. In accordance with the embodiment of figure 11, the curls 11' of the helical serpentine band 113 are not connected with each other by bridges 12 but only by threads 13. In accordance with other possible embodiments, the curls 11' can be also connected with each other by bridges 12.

. In accordance with the embodiment of the stent 1 according to the invention represented in figure 14, at least some of the loops 111 to which a thread 13 is associated, comprise grasping enhancers 115. The grasping enhancers 115 are geometric alterations of the loop made so to have more solid and secure grasping of the thread 13. on the loop 111.

. In accordance with the embodiment represented in figure 14, the grasping enhancers 115 comprise a slot 116 in which the thread 13 is made to pass.

. The grasping enhancers 115 are therefore intended to give place to a form coupling between the loop 111 and the respective thread 13.

. The form coupling can be obtained on a macroscopic scale, as in the examples listed above, or on a more reduced scale. The form coupling can be obtained for example by means of surface grooves of the loop 111 or by means of a high porosity of the same. This could be useful for gluing the thread.

. The form coupling therefore ensures that the grasping of the thread 13 on the serpentine band 11 is more effective and reliable.

. Advantageously, the serpentine bands 11 and the bridges 12, when said stent 1 is of self-expandable type, are in superelastic material. In accordance with a different embodiment, the serpentine bands 11 and the bridges 12 are in a hardened pseudo-elastic material.

. In other words, it is possible to use a material which is in austenitic state at room temperature (i.e. it has a high temperature of end transformation into austenite Af: less than 15°C) when annealed, to which a sufficient hardening treatment followed, for example greater than 30%, which permits having an elastic deformation recovery of 3%-4% or greater. Preferably, a hardening treatment is applied equal to 50%. For simplicity, the above identified material will be referred to below with the expression "superelastic material".

. In accordance with one embodiment, said serpentine bands 11 and said bridges 12 are in a so-called shape memory material.

. Advantageously, said serpentine bands 11 and said bridges 12 are in Nitinol, or Nickel and Titanium - based alloy, for example with nominal percentage by weight of Nickel of 55.8%.

. For example, it is possible to use a material having austenite-martensite phase transition in which, if in annealed or stretched state, during a heating thereof, the high temperature of end transformation into austenite Af is less than 15°C. For simplicity, the above identified alloy will be referred to below with the expression "Nitinol".

. Advantageously, the serpentine bands 11 and the bridges 12, when said stent is of balloon-expandable type, are in stainless steel.

. For example, it is possible to employ a stainless steel of the type classified as AISI 316 L according to the standards of the American Iron and Steel Institute. This alloy of stainless steel has the following standard chemical weight composition: Carbon 0.035%, Phosphorus 0.04%, Sulphur 0.03%, Manganese 2%, Silicon 0.75%, Chromium 16-18%, Nickel 10-15%, Molybdenum 2-3% and Iron to balance. For simplicity, the above identified alloy will be referred to below with the expression "stainless steel".

. Advantageously, the serpentine bands 11 and the bridges 12, when said stent is of balloon-expandable type, are in a non-magnetic alloy of Nickel-Cobalt-Chromium-Molybdenum for surgical implants.

. For example, it is possible to employ an alloy of the type classified as UNS R30035 according to the Unified Numbering System for Metals and Alloys. Such alloy has the following standard composition: Carbon maximum 0.025%, Phosphorus max 0.015%, Sulphur max 0.01%, Manganese max 0.15%, Silicon max 0.15%, Chromium 19-21%, Nickel 33-37%, Molybdenum 9-11%, Titanium max 1%, Boron max 0.01%, Iron max 1% and Cobalt to balance.

. An alloy of this type is commercialised with the name "Carpenter MP35N" which is a trademark of SPS Technologies, Inc. For simplicity, the above identified alloy will be referred to below, with the expression "Chromium-Cobalt alloy".

. In accordance with one embodiment, the serpentine bands 11 and the bridges of said stent 1 are obtained from the cutting of a tubular element, preferably by means of laser cutting.

. According to one possible embodiment, the serpentine bands 11 and the bridges 12 are made integrally from a tubular element by means of cutting, for example laser cutting.

. The materials described up to now with which the serpentine bands 11 and the bridges 12 are made according to the invention are enduring materials. In other words, the serpentine bands 11 and the bridges 12 according to the invention made of superelastic material, in Nitinol, in stainless steel or in Chromium-Cobalt alloy remain nearly unaltered in their dimensions and in the geometries during the operation life in the vessel or duct in which they have been implanted.

. The threads 13 can be made of an enduring material or of a material which is commonly defined as biodegradable, bioerodable or preferably bioabsorbable. In other words, the bioabsorbable material with which each thread 13 is made has the property of dissolving itself in the natural contents of the vessel or duct in which the stent is placed (for example in the blood contained in the vessels) The phenomenon which leads the bioabsorbable material to dissolve itself can be of chemical, electrochemical or physical nature according to the type of material used.

. In accordance with one embodiment of the invention, the thread 13 or the filaments which compose it are made with enduring polymers, such as for example polyamide (PA) and/or polytetrafluoroethylene (PTFE). Such polymers are available on the market with the commercial names of Nylon and Teflon, respectively.

. In accordance with one embodiment of the invention, the thread 13 or filaments which compose it are made with a bioabsorbable polymer. Bioabsorbable polymers particularly adapted for use in the present invention are: PDLA or poly-(D-lactic acid), PLLA or poly-(L-lactic acid), PGA or poly-(glycolic acid).

. Further bioabsorbable polymers adapted for use are the following: poly-caprolactone, poly-(lactide-co-glycolide), poly-(ethylene-vinyl acetate), poly-(hydroxybutyrate-co-valerate), poly-dioxanone, poly-orthoester, poly-anhydride, poly-(glycolic acid-co-trimethylene carbonate), poly-phosphoester, poly-phosphoester urethane, poly(amino acids), cyanoacrylates, poly-(trimethylene carbonate), poly-(iminocarbonate), copoly-(ether-esters) (e.g. PEO/PLA), poly-alkylene oxalates, poly-phosphazenes and biomolecules such as fibrin, fibrinogen, cellulose, starch, collagen, hyaluronic acid, poly-N-alkylacrylamides, poly-depsi-peptide carbonate, and poly-ethylene-oxide based ploy-esters.

. The threads 13 in bioabsorbable polymer can be produced by means of the typical working technologies of this type of polymer. For example, the polymer thread 13 and/or filaments can advantageously be produced, in a known manner, by means of one of the different types of extrusion spinning (wet, dry, in melted state or in gel) or by means of any other technological process which permits satisfying specific needs.

. In a known manner, the structure of the thread 13 can be monofilament or, starting from a plurality of filaments, it can be intertwined or twisted, with or without outer covering.

. The connection between the thread 13 in bioabsorbable polymer and the serpentine band 11 in enduring material can be obtained in various modes.

. One connection form comprises a knot 130 carried out with the thread 13 around a section of a serpentine band 11, independently from the presence of grasping enhancers 115.

. Another connection form comprises a winding 131 executed with the thread 13 around a section of a serpentine band 11, without forming an actual knot 130.

. Several examples of connection by means of knots 130 or windings 131 are schematically represented in the figures 12.

. Finally, a further connection form (see for example figure 12.c) comprises a gluing 132 of the thread 13 on the serpentine band 11. The polymer used for the gluing 132 can be the same with which the thread is made or another of the abovementioned bioabsorbable polymers, according to specific needs.

. Practically speaking, a preferred connection form of a thread 13 to a stent 1 comprises a mixed use of the above-described connection forms. For example, it is possible to knot the thread 13 at a first serpentine band 11.a (proximal end) and then wind it or paste it on the subsequent serpentine bands 11.b, 11.c, etc. without additional knots, but on the last serpentine band (distal end).

. In accordance with some embodiments, the threads 13 are made with bioabsorbable metal materials.

. In accordance with one possible embodiment, the thread 13 is made with a Magnesium alloy.

. For example, it is possible to employ an alloy of the type classified as UNS M18430 according to the Unified Numbering System for Metals and Alloys. Such alloy has the following composition standard: Yttrium 3.7-4.3%, Rare Earths 2.4-4.4% (the Rare Earths consist of Neodymium 2.0-2.5%, the rest being heavy Rare Earths, mainly Ytterbium, Erbium, Dysprosium and Gadolinium), Zirconium min 0.4%, and Magnesium to balance.

. One alloy of this type is commercialised with the name "Elektron WE43", property of Magnesium Elektron of Manchester, UK. For simplicity, the above-identified alloy will be referred to below with the expression "Magnesium alloy".

. The threads 13 in Magnesium alloy can be made by means of any one of the typical working technologies of this alloy type. For example, the threads 13 in Magnesium alloy can be advantageously made by means of drawing, by means of extrusion, by means of hot or cold moulding, by means of sintering, by means of laser working or by means of any other technological process which permits satisfying the specific needs.

. The connection between the Magnesium alloy thread 13 and the serpentine band 11 can be obtained, independently from the presence of the grasping enhancers 115, for example by means of welding or gluing, or by intertwining the thread between the various serpentine bands, according to specific needs. The welding can be carried out with a protective atmosphere technology (for example with TIG technology, Tungsten Inert Gas). The polymer used as glue can be one of the bioabsorbable polymers listed above.

. In accordance with one possible embodiment, the thread 13 is made with a binary mixture of Calcium Oxide (CaO) and Phosphorus Pentoxide ($P_2O_5$).

. For example, it is possible to employ a binary mixture with 5-50% Calcium Oxide (CaO) and 50-95% Phosphorus Pentoxide ($P_2O_5$). Preferably, the binary mixture is composed of 15-25% Calcium Oxide (CaO) and 65-85% Phosphorus Pentoxide ($P_2O_5$). Such binary mixture can also contain small quantities of Calcium Fluoride ($CaF_2$), water ($H_2O$) and other oxides of Magnesium, Zinc, Strontium, Sodium, Potassium, Lithium or Aluminium.

. For simplicity, the above-indicated mixture will be referred to below with the expression "Calcium-Phosphorus mixture".

. The threads 13 in Calcium-Phosphorus mixture can be made by means of any one the typical working technologies of this material type. For example, the threads 13 in Calcium-Phosphorus mixture can be advantageously made by means of drawing, extrusion, melting, hot moulding or any other technological process which permits satisfying specific needs.

. The connection between the thread 13 in Calcium-Phosphorus mixture and the serpentine band 11 can be obtained, independently from the presence of the grasping enhancers 115, for example, by means of welding or gluing, or by intertwining the thread 13 between the various serpentine bands 11, according to the specific needs. The polymer used as glue can be used as a bioabsorbable polymer of those listed above.

. In accordance with some embodiments, a single thread 13 is arranged along the stent 1, preferably along the entire length, or rather along its entire longitudinal length. The thread 13 is a structure which has a predominantly axial extension and which joins more than two serpentine bands 11.

. In accordance with other embodiments, a plurality of threads 13 is present, as shown schematically in figures 1-3 and 6.

. In accordance with one embodiment, an end serpentine band (for example the serpentine 11.a placed at the distal end) comprises a marker made in radiopaque material.

. In fact, when the serpentine bands 11 of the stent 1 are made, for example, in superelastic material or in Nitinol and the threads 13 are made, for example, in polymer material, the stent would be entirely invisible to the radioscopy.

. A stent which is not visible to the radioscopy poses very serious problems to the operator who must implant it in a patient using the conventional - radioscopic apparatus to follow the movements and positioning of the stent along the vessels of the patient.

. The radiopaque material with which the marker is made can be chosen from Tantalum, Gold, Platinum, Tungsten or other materials suitable for such purpose.

. According to one possible embodiment, both serpentine bands placed at the distal and proximal end of the stent 1, i.e. and the first and the last serpentine band, respectively comprise at least one radiopaque marker.

. Due to the proposed stent, it is possible to execute endoluminal operations in tortuous ducts or vessels and ensure at the same time, with expanded prosthesis, an optimal and uniform support of the wall of the treated vessel.

. In accordance with one embodiment of the stent 1 according to the invention, the thread 13 made of bioabsorbable material is adapted to release a drug in a controlled manner and prolonged over time.

. The threads 13 can be previously treated so to be porous. In the pores of the bioabsorbable material, a pharmacologically active substance can be inserted which is adapted for the treatment of the zone in which the stent 1 is implanted.

. With this particular embodiment of the invention, in a known manner, there is the controlled release of the drug, prolonged over time. Thus an important pharmacological contribution is obtained in the acute phase of the treatment carried out by means of the stent 1.

. Analogously to the action of the possible drug set in the pores of the bioabsorbable material, it should be noted how the magnesium itself with which the bioabsorbable threads 13 can be obtained has positive effects on the containment of the cellular proliferation in the zone where the stent 1 is implanted.

. Some important mechanical characteristics of the enduring and bioabsorbable metal materials described above are provided below.

| | | Stainless (AISI316L) | Cr-Co (MP35N) | NiTinol | Magnesium Alloy |
|---|---|---|---|---|---|
| E | Elastic modulus, GPa | 193 | 233 | 90 | 44 |
| $\sigma_{0.2}$ | Yield strength, MPa | 340 | 414 | - | 178 |
| $\sigma_r$ | Breaking strength, MPa | 670 | 930 | 1400 | 250 |

. Alongside the characteristics of the materials listed above, several characteristics of the stent should also be underlined, and how much they are dependent both on the material and on the utilised geometry.

. One extremely important characteristic of the stent is the radial force. It describes the capacity of the stent to resist circumferential loads. It is definable as the radial force which the stent is capable of exerting inside a vessel once it has been correctly implanted therein.

. Such characteristic is extremely important, since it determines the capacity of the stent to keep open the treated vessel. The radial force depends on the geometry and above all on the elastic modulus E of the employed material. The higher the value of the elastic modulus, the greater the radial force which can be obtained by the stent.

. A further important characteristic in the evaluation of a balloon-expandable stent is called 'recoil'. The recoil is, in percentage, the elastic return of the stent following the expansion. In fact, during the expansion, the stent is over-expanded to take into account the inevitable elastic return.

. The recoil of a stent can be defined as follows:

$$\text{recoil} = \frac{(\text{over-expanded diameter} - \text{expanded diameter}) * 100}{\text{over-expanded diameter}}$$

. The lower the recoil, the lower the over-expansion necessary to effectively implant the stent, and consequently the lower risk of possible vessel dissections.

. A low recoil, in addition to an appropriate geometry of the stent, can be obtained due to a high elastic modulus E and to a not overly high yield strength $\sigma_{0.2}$.

. In view of these considerations and the characteristics of the materials reported in the table, it is immediately possible to understand how a stent made, for example, entirely in Magnesium alloy cannot ensure a considerably radial force, since the elastic module of the Magnesium alloy is relatively moderate.

. The present invention, by permitting the use of different materials inside the same endoluminal prosthesis, permits the designer to balance the characteristics of one material with that of another.

. One is thus able to obtain, for example stents made with wide use of magnesium threads, which have however an acceptable radial force due to the stainless steel tubular body 10.

. In view of that described above, it will now be clear to the person skilled in the art how an endoluminal prosthesis according to the invention resolves the problems set forth with reference to the prior art.

. In particular, now it will be clear how the stent 1 described above according to the invention can resolve the problem of sustaining the vessel wall more immediately after the implant, and then reducing the effect over a long period.

. In fact, immediately after the implant of the stent, both the serpentine band and the bridges and threads contribute to supporting the walls of the vessel. Subsequently, once the acute phase is terminated, the bioabsorbable threads are dissolved, for example in the blood, and there remain only the parts in enduring material (the serpentine bands and the bridges, if present) therefore limiting the stress on the wall.

. The presence of the thread 13 at the time of the stent implant inhibits the jumping ahead phenomenon of the stent at the time of release. In fact, the thread 13 blocks the stent 1 from suddenly expanding at the time of the removal from the sheath.

. At the same time, the presence of the threads 13 in the first phases of the stent implant and in the immediately following phases ensures an optimal positioning of the stent in its entirety and ensures that the single serpentine bands assume a correct position with respect to each other.

. The embodiments of the stent in which the bands 11 are exclusively connected by threads 13 and which therefore do not have bridges 12 resolve the problem of the potential fatigue breaking of the bridges themselves.

. The embodiment of figure 7 in which each loop is connected to the adjacent loop permits the operator, during the operation, to adjust the position of the stent along the vessel wherein it is to be implanted. This operation is made possible by the particular conformation in which a bridge 12 or a thread 13 is provided for each loop 111. Such conformation permits perfectly connecting the serpentine bands which had already been uncovered by the withdrawal of the sheath with the serpentine bands which are still covered by the sheath. This characteristic permits the operator to re-push the sheath ahead along the catheter, and along the stent 1, so to close the serpentine bands which were previously open.

. The operation of closing the stent 1 and repositioning it is of particular use. The steps of insertion and implantation of the stent 1 are extremely delicate. The smallest positioning error of the stent can lead to very serious consequences, even requiring the need for emergency surgery on the patient to remove a stent opened in an erred position.

. The re-push operation of the sheath along the catheter and along the stent 1 is not possible with traditional stents. In fact the loops 111 of the serpentine bands which have just been uncovered by the withdrawal of the sheath tend to exit from the ideal profile of the stent so to form steps which block the opposite movement of the sheath along the stent 1.

. The presence of a thread 13 for each of the loops 111 was made possible by the fact that such threads 13 can be made of bioabsorbable material. In a stent of traditional type, completely made of enduring material, it would not be possible to achieve such configuration due to the excessive quantity of metal which would be found on the surface of the expanded stent. Indeed, the surface covered by metal must not exceed $14 \div 15$ % of the total surface.

. In accordance with one embodiment, schematically illustrated in figure 12.g, the stent 1 also comprises at least one thread 13' which has a length greater than double the catheter employed for the positioning of the stent in the duct inside the human body.

. In accordance with such embodiment, the thread 13' is wound without any knot around a serpentine band of the stent 1. When the stent is loaded on the catheter, the thread 13' is passed along its entire length so that both of its ends are reachable at the proximal end of the catheter itself.

. Using such embodiment of the present invention, the operator can apply a traction action on the stent and can therefore maintain greater control during the delicate positioning step. At the end of such step, the thread 13' can be unthreaded by pulling on one of the two ends.

. It is clear that variants and/or additions to what described and illustrated above can be provided.

. The number of threads 13, serpentine bands 11, arms 110 and loops 111 can vary with respect to what described and illustrated. Also the form of the serpentine bands can vary.

. In general, all characteristics described above in relation to specific possible embodiments can be made independent from each other.

. A person skilled in the art, in order to satisfy contingent and specific needs, can make numerous modifications and adaptations to the preferred embodiments of the endoluminal stent described above, as well as substitutions of elements with functionally equivalent elements, without however departing from the scope of the following claims.

**Claims**

1. Endoluminal prosthesis (1) or stent comprising a tubular body (10) suitable to be brought from a contracted condition to an expanded condition, said tubular body (10) extending along a longitudinal axis (X-X), said tubular body (10) comprising a plurality of bands (11, 11') comprising serpentine bands (11.a, 11.b, 11.c),
said plurality of bands (11) are connected to each other by integral bridges (12) made in one piece with the bands (11)

said bridges (12) are capable of offering resistance to forces applied along the X-X axis
**characterized in that**
more than two serpentine bands (11.a, 11.b, 11.c) are connected with each other by means of a single thread (13; 13'), having a winding (131) executed with the thread (13; 13') around a serpentine band (11.a; 11.b; 11.c).

2. Stent (1) according to the preceding claim wherein said stent (1) is of the self-expandable type or is of the balloon-expandable type and/or wherein said bands comprise serpentine bands (11) which define paths which are closed on themselves and wherein said bands (11, 11') are extended along a substantially circumferential direction (C) or sway little therefrom and wherein each of said serpentine bands comprise arms (110) and loops (111) which connect two subsequent arms (110) to form a meandering path and/or the bands (11) of the stent (1) are connected to each other also by integral bridges (12) made in one piece with the bands (11) and/or the bands of the stent (1) are composed of curls (11') of a single, long helical serpentine band (113) and/or said bands (11) and said bridges (12) are made of an enduring material chosen from the group comprising: superelastic material, Nitinol, stainless steel and Chromium-Cobalt alloy.

3. Stent (1) according to any one of the preceding claims wherein said at least one thread (13) is connected to at least two bands (11.a, 11.b) or to at least two adjacent bands (11.a, 11.b).

4. Stent (1) according to any one of the preceding claims wherein said at least one thread (13) is connected to at least two non-adjacent bands (11.a, 11.c).

5. Stent (1) according to any one of the preceding claims wherein said thread (13) is an elongated and extremely flexible element defining its own axis, wherein the characteristic dimensions of any cross section of the thread (13) transverse to its own axis are negligible with respect to the third dimension along the axis.

6. Stent (1) according to the preceding claim, where said thread (13) is composed of a single filament or wherein said thread (13) is composed of a plurality of filaments which are intertwined or twisted with each other so to remain assembled together and/or said thread (13) comprises an outer covering.

7. Stent (1) according to any one of the preceding claims wherein said thread (13) has mechanical characteristics such to permit its reacting in a significant manner only to traction strains along its axis.

8. Stent (1) according to any one of the preceding claims wherein said thread (13) has an extension oriented in a direction substantially parallel to the X-X axis of the stent (1) and/or said thread (13) has a direction tilted an angle equal to $\pm\alpha$ with respect to the X-X axis of the tubular body 10 and/or said thread (13) is prevalently arranged on an outer surface of the stent (1).

9. Stent (1) according to any one of the preceding claims, wherein said thread (13) has an extension oriented, in addition to in an axial direction, also partially in a circumferential direction, so to obtain a helical progression along the stent (1).

10. Stent (1) according to any one of the preceding claims wherein the bands (11) of the stent (1) are connected to each other exclusively by threads (13).

11. Stent (1) according to any one of the preceding claims wherein between adjacent bands (11.a, 11.b) a plurality of threads (13) is comprised and or every single loop (111) of every single serpentine band (11.b), is connected to the respective loop (111) of the adjacent serpentine band (11.a, 11.c) by means of a thread (13) or by means of a bridge (12) and/or the threads (13) between at least two adjacent serpentine bands (11.a, 11.b) are parallel to each other.

12. Stent (1) according to any one of the preceding claims comprising sections (120) comprising in turn serpentine bands (11) joined together by bridges (12), said sections (120) being connected to each other exclusively by threads (13) and not by bridges (12).

13. Stent (1) according to the preceding claim wherein the number of serpentine bands (11) for each section (120) increases along the X-X axis from the proximal end towards the centre of the stent (1), and once the maximum has been reached in the central section (120), decreases along the X-X axis from the centre of the stent (1) towards the distal end.

14. Stent (1) according to claim 11 wherein the threads (13) have different lengths, each thread covering the central portion of the stent (1).

15. Stent (1) according to any one of the preceding claims wherein at least some of the loops (111) to which the thread (13) is associated comprise grasping enhancers (115) adapted to make the grasping of the thread (13) on the loop (111) more solid and secure.

16. Stent (1) according to the preceding claim wherein the grasping enhancers (115) comprise a slot (116) in which the thread (13) can be passed.

17. Stent (1) according to claim 15, wherein the grasping enhancers (115) comprise grooves or a high porosity of the surface of the loop (111).

18. Stent (1) according to any one of the preceding claims comprising a thread (13) made of an enduring material chosen from the group comprising: polyamide (PA) and polytetrafluoroethylene (PTFE).

19. Stent (1) according to any one of the preceding claims comprising a thread (13) made of a bioabsorbable material and/or said bioabsorbable material is a polymer selected from the group composed of PDLA or poly-(D-lactic acid), PLLA or poly-(L-lactic acid), and PGA or poly-(glycolic acid) and/or said bioabsorbable material is a polymer selected from the group composed of: poly-caprolactone, poly-(lactide-co-glycolide), poly-(ethylene-vinyl acetate), poly-(hydroxybutyrate-co-valerate), poly-dioxanone, poly-orthoester, poly-anhydride, poly-(glycolic acid-co-trimethylene carbonate), poly-phosphoester, poly-phosphoester urethane, poly(amino acids), cyanoacrylates, poly-(trimethylene carbonate), poly-(iminocarbonate), copoly-(ether-esters) (e.g. PEO/PLA), poly-alkylene oxalates, poly-phosphazenes and biomolecules such as fibrin, fibrinogen, cellulose, starch, collagen, hyaluronic acid, poly-N-alkylacrylamides, poly-depsi-peptide carbonate, and poly-ethylene-oxide based ploy-esters and/or
said bioabsorbable material is a metal material selected from the group composed of Magnesium alloy and Calcium-Phosphorus mixture and/or said bioabsorbable material is adapted to release a drug in a manner controlled and prolonged over time.

20. Stent (1) according to any one of the preceding claims wherein the connection between said thread (13) and said band (11) comprises a knot (130) of the thread (13) around a section of said band (11) and/or the connection between said thread (13) and said band (11) comprises a winding (131) of the thread (13) around a section of said band and/or the connection between said thread (13) and said band (11) comprise a gluing (132) of the thread (13) on a section of said band (11).

21. Stent (1) according to any one of the preceding claims comprising at least one radiopaque marker.

22. Stent (1) according to any one of the preceding claims wherein said thread (13') is over double the length of the catheter employed for positioning the stent (1) and is connected to a serpentine band (11), so that both its ends can be reached at the proximal end of the catheter during catheter use.

23. Stent (1) according to the preceding claim wherein the thread (13') can be unthreaded by pulling on one of the two ends.

24. **Stent (1) according to claim 1, wherein between adjacent serpentine bands (11.a, 11.b, 11.c) a plurality of threads (13, 13') is provided.**

25. Kit comprising a stent (1) in accordance with any one of the preceding claims and a catheter adapted for the positioning of said stent (1) inside a duct.

**Patentansprüche**

1. Endoluminalprothese (1) oder Stent, umfassend einen röhrenförmigen Korpus (10), der dafür geeignet ist, von einem zusammengezogenen Zustand zu einem ausgedehnten Zustand gebracht zu werden, wobei sich der röhrenförmige Korpus (10) längs einer Längsachse (X-X) erstreckt, wobei der röhrenförmige Korpus (10) mehrere Bänder (11, 11') umfasst, die schlangenförmige Bänder (11.a, 11.b, 11.c) umfassen,
wobei die mehreren Bänder (11) durch integrale Brücken (12), die in einem Stück mit den Bändern (11) hergestellt sind, miteinander verbunden sind,
wobei die Brücken (12) dazu in der Lage sind, Kräften, die längs der Achse X-X ausgeübt werden, Widerstand entgegenzusetzen,
**dadurch gekennzeichnet, dass**
mehr als zwei schlangenförmige Bänder (11.a, 11.b, 11.c) mit Hilfe eines einzelnen Strangs (13; 13') miteinander verbunden sind, der eine Windung (131) hat, die mit dem Strang (13; 13') um ein schlangenförmiges Band (11.a, 11.b, 11.c) ausgeführt ist.

2. Stent (1) nach dem vorhergehenden Anspruch, wobei der Stent (1) von dem sich selbst ausdehnenden Typ ist oder von dem ballonausdehnbaren Typ ist und/oder wobei die Bänder schlangenförmige Bänder (11) umfassen, die Bahnen definieren, die auf sich selbst geschlossen sind, und wobei sich die Bänder (11, 11') längs einer im Wesentlichen umlaufenden Richtung (C) erstrecken oder wenig von derselben abweichen und wobei jedes der schlangenförmigen Bänder Arme (110) und Schleifen (111), die zwei aufeinanderfolgende Arme (110) verbinden, umfasst, um eine mäandernde Bahn zu bilden, und/oder die Bänder (11) des Stents (1) miteinander ebenfalls durch integrale Brücken (12) verbunden sind, die in einem Stück mit den Bändern (11) hergestellt sind, und/oder die Bänder des Stents (1) aus Windungen (11') eines einzelnen, langen spiralförmigen schlangenförmigen Bandes (113) bestehen und/oder die Bänder (11) und die Brücken (12) aus einem beständigen Werkstoff hergestellt sind, der ausgewählt ist aus der Gruppe, die Folgendes umfasst: superelastischen Werkstoff, Nitinol, rostfreien Stahl und eine Chrom-Kobalt-Legierung.

3. Stent (1) nach einem der vorhergehenden Ansprüche, wobei der wenigstens eine Strang (13) mit wenigstens zwei Bändern (11.a, 11.b) oder mit wenigstens zwei benachbarten Bändern (11.a, 11.b) verbunden ist.

4. Stent (1) nach einem der vorhergehenden Ansprüche, wobei der wenigstens eine Strang (13) mit wenigstens zwei nicht benachbarten Bändern (11.a, 11.c) verbunden ist.

5. Stent (1) nach einem der vorhergehenden Ansprüche, wobei der Strang (13) ein längliches und äußerst flexibles Element ist, das seine eigene Achse definiert, wobei die kennzeichnenden Abmessungen eines beliebigen Querschnitts des Strangs (13) quer zu seiner eigenen Achse in Bezug auf die dritte Dimension längs der Achse unerheblich sind.

6. Stent (1) nach dem vorhergehenden Anspruch, wobei der Strang (13) aus einem einzelnen Filament besteht oder wobei der Strang (13) aus mehreren Filamenten besteht, die miteinander verflochten oder verdrillt sind, so dass sie aneinandergefügt bleiben, und/oder der Strang (13) einen äußeren Überzug umfasst.

7. Stent (1) nach einem der vorhergehenden Ansprüche, wobei der Strang (13) derartige mechanische Eigenschaften hat, dass sein Reagieren auf eine bedeutsame Weise nur auf Zugbeanspruchungen längs seiner Achse ermöglicht wird.

8. Stent (1) nach einem der vorhergehenden Ansprüche, wobei der Strang (13) eine Ausdehnung hat, die in einer Richtung, im Wesentlichen parallel zu der Achse X-X des Stents (1), ausgerichtet ist, und/oder der Strang (13) eine Richtung hat, die in einem Winkel, gleich $\pm\,\alpha$, in Bezug auf die Achse X-X des röhrenförmigen Korpus (10) geneigt ist, und/oder der Strang (13) vorherrschend auf einer Außenfläche des Stents (1) angeordnet ist.

9. Stent (1) nach einem der vorhergehenden Ansprüche, wobei der Strang (13) eine Ausdehnung hat, die, außer in einer axialen Richtung, ebenfalls teilweise in einer umlaufenden Richtung ausgerichtet ist, um so ein spiralförmiges Fortschreiten längs des Stents (1) zu erreichen.

10. Stent (1) nach einem der vorhergehenden Ansprüche, wobei die Bänder (11) des Stents (1) ausschließlich durch Stränge (13) miteinander verbunden sind.

11. Stent (1) nach einem der vorhergehenden Ansprüche, wobei zwischen benachbarten Bändern (11.a, 11.b) mehrere Stränge (13) enthalten sind und/oder jede einzelne Schleife (111) jedes einzelnen schlangenförmigen Bandes (11.b) mit Hilfe eines Strangs (13) oder mit Hilfe einer Brücke (12) mit der jeweiligen Schleife (111) des benachbarten schlangenförmigen Bandes (11.a, 11.c) verbunden ist und/oder die Stränge (13) zwischen wenigstens zwei benachbarten schlangenförmigen Bändern (11.a, 11.b) parallel zueinander sind.

12. Stent (1) nach einem der vorhergehenden Ansprüche, der Sektionen (120) umfasst, die wiederum schlangenförmige Bänder (11) umfassen, die durch Brücken (12) miteinander verbunden sind, wobei die Sektionen (120) ausschließlich durch Stränge (13) und nicht durch Brücken (12) miteinander verbunden sind.

**13.** Stent (1) nach dem vorhergehenden Anspruch, wobei die Anzahl von schlangenförmigen Bändern (11) für jede Sektion (120) längs der Achse X-X von dem proximalen Ende zu der Mitte des Stents (1) hin zunimmt, und sobald in der mittleren Sektion (120) das Maximum erreicht worden ist, längs der Achse X-X von der Mitte des Stents (1) zu dem distalen Ende hin abnimmt.

**14.** Stent (1) nach Anspruch 11, wobei die Stränge (13) unterschiedliche Längen haben, wobei jeder Strang den mittleren Abschnitt des Stents (1) abdeckt.

**15.** Stent (1) nach einem der vorhergehenden Ansprüche, wobei wenigstens einige der Schleifen (111), mit denen der Strang (13) verknüpft ist, Griffverbesserer (115) umfassen, um das Greifen des Strangs (13) an der Schleife (111) fester und sicherer zu machen.

**16.** Stent (1) nach dem vorhergehenden Anspruch, wobei die Griffverbesserer (115) einen Schlitz (116) umfassen, in dem der Strang (13) hindurchgeführt werden kann.

**17.** Stent (1) nach Anspruch 15, wobei die Griffverbesserer (115) Rillen oder eine hohe Porosität der Schleife (111) umfassen.

**18.** Stent (1) nach einem der vorhergehenden Ansprüche, der einen Strang (13) umfasst, der aus einem beständigen Werkstoff hergestellt ist, der ausgewählt ist aus der Gruppe, die Folgendes umfasst: Polyamid (PA) und Polytetrafluorethylen (PTFE).

**19.** Stent (1) nach einem der vorhergehenden Ansprüche, der einen Strang (13) umfasst, der aus einem biologisch absorbierbaren Werkstoff hergestellt ist und/oder wobei der biologisch absorbierbare Werkstoff ein Polymer ist, das ausgewählt ist aus der Gruppe, die aus PDLA oder Poly-(D-Milchsäure), PLLA oder Poly-(L-Milchsäure) und PGA oder Poly-(Glykolsäure) besteht, und/oder der biologisch absorbierbare Werkstoff Polymer ist, das ausgewählt ist aus der Gruppe, die Folgendes umfasst: Polycaprolacton, Poly-(Lactid-co-glycolid), Poly-(Ethylen-Vinylacetat), Poly-(Hydroxybutyrat-co-valerat), Polydioxanon, Polyorthoester, Polyanhydrid, Poly-(Glykolsäure-co-trimethylencarbonat), Polyphosphoester, Polyphosphoesterurethan, Poly-(Aminosäuren), Cyanoacrylate, Poly-(Trimethylencarbonat), Poly-(Iminocarbonat), Copoly-(Ether-Ester) (z.B. PEO/PLA), Polyalkylenoxalate, Polyphosphazene und Biomoleküle, wie beispielsweise Fibrin, Fibrinogen, Cellulose, Stärke, Collagen, Hyaluronsäure, Poly-N-Alkylacrylamide Poly-Depsipeptid-Carbonat und Polyester auf Polyethylenoxid-Grundlage, und/oder
der biologisch absorbierbare Werkstoff ein Metallwerkstoff ist, der ausgewählt ist aus der Gruppe, die aus einer Magnesiumlegierung und einem Kalzium-Phosphor-Gemisch besteht, und/oder der biologisch absorbierbare Werkstoff dafür eingerichtet ist, ein Arzneimittel auf eine über die Zeit geregelte und ausgedehnte Weise abzugeben.

**20.** Stent (1) nach einem der vorhergehenden Ansprüche, wobei die Verbindung zwischen dem Strang (13) und dem Band (11) einen Knoten (130) des Strangs (13) um eine Sektion des Bandes (11) umfasst und/oder die Verbindung zwischen dem Strang (13) und dem Band (11) eine Windung (131) des Strangs (13) um eine Sektion des Bandes (11) umfasst und/oder die Verbindung zwischen dem Strang (13) und dem Band (11) eine Verklebung (132) des Strangs (13) an einer Sektion des Bandes (11) umfasst.

**21.** Stent (1) nach einem der vorhergehenden Ansprüche, der wenigstens eine strahlungsundurchlässige Markierung umfasst.

**22.** Stent (1) nach einem der vorhergehenden Ansprüche, wobei der Strang (13') mehr als das Doppelte der Länge des für das Positionieren des Stents (1) eingesetzten Katheters beträgt und mit einem schlangenförmigen Band (11) verbunden ist, so dass seine beiden Enden während der Katheterverwendung an dem proximalen Ende des Katheters erreicht werden können.

**23.** Stent (1) nach dem vorhergehenden Anspruch, wobei der Strang (13') durch das Ziehen an einem der zwei Enden herausgefädelt werden kann.

**24.** Stent (1) nach Anspruch 1, wobei zwischen benachbarten schlangenförmigen Bändern (11.a, 11.b, 11.c) mehrere Stränge (13, 13') bereitgestellt werden.

**25.** Besteck, das einen Stent (1) nach einem der vorhergehenden Ansprüche und einen Katheter, der für das Positionieren des Stents (1) innerhalb eines Ganges eingerichtet ist, umfasst.

**Revendications**

**1.** Prothèse endoluminale (1) ou stent comprenant un corps tubulaire (10) apte à être amené d'une position contractée à une position dilatée, ledit corps tubulaire (10) s'étendant le long d'un axe longitudinal (X-X), ledit corps tubulaire (10) comprenant une pluralité de bandes (11, 11') comprenant des bandes en serpentin (11.a, 11.b, 11.c),
ladite pluralité de bandes (11, 11') étant connectées entre elles par des ponts intégraux (12) constitués d'une pièce avec les bandes (11),
lesdits ponts (12) étant aptes à offrir une résistance aux forces appliquées le long de l'axe X-X,
**caractérisée en ce que**
plus de deux bandes en serpentin (11.a, 11.b, 11.c) sont connectées entre elles au moyen d'un seul fil (13 ; 13') présentant un enroulement (131) réalisé avec le fil (13 ; 13') autour d'une bande en serpentin (11.a, 11.b, 11.c).

**2.** Stent (1) selon la revendication précédente, ledit stent (1) étant de type auto-expansible ou étant de type ballon expansible et/ou lesdites bandes comprenant des bandes en serpentin (11) qui définissent des parcours qui sont fermés sur eux-mêmes et lesdites bandes (11 ; 11') s'étendant suivant un sens sensiblement circonférentiel (C) ou allant et venant légèrement depuis celui-ci et chacune desdites bandes en serpentin (110) et boucles (111) connectant deux bras consécutifs (110) pour former un parcours sinueux et/ou les bandes (11) du stent (1) étant connectées entre elles par deux ponts intégraux (12) constitués d'une pièce avec les bandes (11) et/ou les bandes du stent (1) étant composées d'ondulations (11') d'une seule longue bande hélicoïdale en serpentin (113) et/ou lesdites bandes (11) et lesdits ponts (12) étant faits d'un matériau résistant choisi dans le groupe composé de : matériau super-élastique, Nitinol, acier inox et alliage de chrome-cobalt.

**3.** Stent (1) selon l'une quelconque des revendications précédentes, ledit au moins un fil (13) étant connecté à au moins deux bandes (11.a, 11.b) ou à au moins deux bandes adjacentes (11.a, 11.b).

**4.** Stent (1) selon l'une quelconque des revendications précédentes, ledit au moins un fil (13) étant connecté à au moins deux bandes non adjacentes (11.a, 11.c).

**5.** Stent (1) selon l'une quelconque des revendications précédentes, ledit au moins un fil (13) étant un élément allongé et extrêmement souple définissant son propre axe, les dimensions caractéristiques d'une section transversale quelconque du fil (13) transversalement par rapport à son propre axe étant négligeables par rapport à la troisième dimension le long de l'axe.

**6.** Stent (1) selon la revendication précédente, ledit fil (13) étant composé d'un seul filament ou ledit fil (13) étant composé d'une pluralité de filaments qui sont entrelacés ou tordus entre eux de manière à rester assemblés ensemble et/ou ledit fil (13) comprenant un recouvrement extérieur.

**7.** Stent (1) selon l'une quelconque des revendications précédentes, ledit fil (13) ayant des caractéristiques mécaniques de nature à lui permettre de réagir de manière significative seulement aux contraintes de traction le long de son axe.

**8.** Stent (1) selon l'une quelconque des revendications précédentes, ledit fil (13) ayant une extension orientée dans un sens sensiblement parallèle à l'axe X-X du stent (1) et/ou ledit fil (13) ayant un sens incliné suivant un angle égal à $\pm\alpha$ par rapport à l'axe X-X du corps tubulaire (10) et/ou ledit fil (13) étant majoritairement disposé sur une surface extérieure du stent (1).

**9.** Stent (1) selon l'une quelconque des revendications précédentes, ledit fil (13) ayant une extension orientée également partiellement, outre dans un sens axial, dans un sens circonférentiel de manière à obtenir une progression hélicoïdale le long du stent (1).

**10.** Stent (1) selon l'une quelconque des revendications précédentes, les bandes (11) du stent (1) étant connectées entre elles exclusivement par des fils (13).

**11.** Stent (1) selon l'une quelconque des revendications précédentes, une pluralité de fils (13) étant compris entre les bandes adjacentes (11.a, 11.b) et/ou chaque boucle individuelle (111) de chaque bande en serpentin (11.b) étant connectée à la boucle respective (11) de la bande en serpentin adjacente (11.a, 11.c) au moyen d'un fil (13) ou au moyen d'un pont (12) et/ou les fils (13) situés entre au moins deux bandes en serpentin adjacentes (11.a, 11.b) étant parallèles entre eux.

12. Stent (1) selon l'une quelconque des revendications précédentes, comprenant des sections (120) comprenant à leur tour des bandes en serpentin (11) jointes entre elles par des ponts (12), lesdites sections (120) étant connectées entre elles exclusivement par des fils (13) et non pas par des ponts (12).

13. Stent (1) selon la revendication précédente, le nombre de bandes en serpentin (11) pour chaque section (120) augmentant le long de l'axe X-X depuis l'extrémité proximale vers le centre du stent (1) et, une fois que le maximum a été atteint dans la section centrale (120), diminuant le long de l'axe X-X depuis le centre du stent (1) vers l'extrémité distale.

14. Stent (1) selon la revendication 11, les fils (13) ayant des longueurs différentes, chaque fil couvrant la partie centrale du stent (1).

15. Stent (1) selon l'une quelconque des revendications précédentes, au moins certaines des boucles (111) auxquelles le fil (13) est associé comprenant des renforçateurs de saisie (115) aptes à rendre la saisie du fil (13) sur la boucle (111) plus solide et sûre.

16. Stent (1) selon la revendication précédente, les renforçateurs de saisie (115) comportant une fente (116) dans laquelle le fil (3) peut être passé.

17. Stent (1) selon la revendication 15, les renforçateurs de saisie (115) comportant des gorges de haute porosité ou présentant une porosité élevée sur la surface de la boucle (111).

18. Stent (1) selon l'une quelconque des revendications précédentes, comprenant un fil (13) fait d'un matériau résistant choisi dans le groupe composé de : polyamide (PA) et polytétrafluoroéthylène (PTFE).

19. Stent (1) selon l'une quelconque des revendications précédentes, comprenant un fil (13) fait d'un matériau bioabsorbable et/ou ledit matériau bioabsorbable étant un polymère sélectionné dans le groupe composé de PDLA ou poly-(D-acide lactique), PLLA ou poly-(L-acide lactique) et PGA ou poly-(acide glycolique) et/ou ledit matériau bioabsorbable étant un polymère sélectionné dans le groupe composé due : poly-caprolactone, poly-(lactide-co-glycolide), poly-(éthylène-vinyle-acétate), poly-(hydroxybutyrate-co-valérate), poly-dioxanone, poly-orthoester, poly-anhydride, poly-(acide glycolique-co-triméthylène carbonate), poly-phosphoester, poly-phosphoester uréthane, poly(acides aminés), cyanoacrylates, poly-(triméthylène carbonate), poly-(iminocarbonate), copoly-(éther-esters) (comme le PEO/PLAN), poly-alkylène oxalates, poly-phosphazènes et biomolécules comme la fibrine, le fibrinogène, la cellulose, l'amidon, le collagène, l'acide hyaluronique, les poly-N-alkylacrylamides, le poly-depsi-peptide carbonate et les polyesters à base de polyéthylène-oxyde) et/ou ledit matériau bioabsorbable étant un matériau métallique sélectionné dans le groupe composé de l'alliage de magnésium et du mélange de calcium-phosphore et/ou ledit matériau bioabsorbable étant apte à diffuser un médicament d'une manière contrôlée et prolongée dans le temps.

20. Stent (1) selon l'une quelconque des revendications précédentes, la connexion entre ledit fil (13) et ladite bande (11) comprenant un noeud (130) du fil (13) autour d'une section de ladite bande (11) et/ou la connexion entre ledit fil (13) et ladite bande (1) comprenant un enroulement (131) du fil (13) autour d'une section de ladite bande et/ou la connexion entre ledit fil (13) et ladite bande (1) comprenant un collage (132) du fil (13) sur une section de ladite bande (1).

21. Stent (1) selon l'une quelconque des revendications précédentes, comprenant au moins un marqueur radio-opaque.

22. Stent (1) selon l'une quelconque des revendications précédentes, ledit fil (13') ayant plus du double de la longueur du cathéter employé pour positionner le stent (1) et étant connecté à une bande en serpentin (11) de manière à ce que ses deux extrémités puissent être atteintes à l'extrémité proximale du cathéter pendant l'utilisation du cathéter.

23. Stent (1) selon la revendication précédente, le fil (13') pouvant être défait en tirant sur une des deux extrémités.

24. Stent (1) selon la revendication 1, une pluralité de fils (13, 13') étant prévus entre des bandes en serpentin adjacentes (11.a, 11.b, 11.c).

25. Kit comprenant un stent (1) selon l'une quelconque des revendications précédentes et un cathéter apte à positionner ledit stent (1) dans un conduit.

**Fig. 1**

**Fig. 2**

**Fig. 3**

Fig. 4

Fig. 5

Fig. 6

Fig. 12.a

Fig. 12.b

Fig. 12.c

Fig. 12.d

Fig. 12.e

**Fig. 12.f**

**Fig. 12.g**

**Fig. 13**

**Fig. 14**

FIG.7

FIG.8

EP 2 124 847 B1

FIG.9

EP 2 124 847 B1

FIG.10

FIG.11

**EP 2 124 847 B1**

**Patent documents cited in the description**

- WO 2006034062 A
- WO 2006058206 A
- WO 2008030488 A
- WO 9413268 A